# EUROPEAN PATENT APPLICATION

(11) **EP 2 113 287 A2**
(43) Date of publication of application: **04.11.2009**
(21) Application number: 09006013.8
(22) Date of filing: 30.04.2009
(51) Int. Cl.: A61Q 7/00, A61K 8/64

(54) **Composition for hair**

(30) Priority: 02.05.2008 JP 2008120287
(71) Applicant: FUJIFILM Corporation, Minato-ku Tokyo (JP)
(72) Inventor: Kanazawa, Katsuhiko, Kanagawa 258-8538 (JP)
(74) Representative: HOFFMANN EITLE

(57) **Abstract**

It is an object of the present invention to provide a composition for hair which comprises a hydrophobic ingredient although it has an extremely low alcohol concentration, and which has a low viscosity. The present invention provides a composition for hair having a viscosity of 30 P or less, which comprises 1% to 50% by weight of alcohol and which further comprises a hydrophobic hair active ingredient.

## Description

### Technical Field

The present invention relates to a composition for hair, which comprises 1% to 50% by weight of alcohol, and which further comprises a hydrophobic hair active ingredient.

### Background Art

There are a large number of hydrophobic substances among compounds having nutritive, physiological, and pharmacological functions. When such hydrophobic substance is orally or transdermally taken into a body as an ingredient of foods or pharmaceutical products, it is desired that the hydrophobic substance is solubilized in an aqueous solution that can easily be taken into the body. However, when a hydrophobic substance is solubilized in an aqueous solution, it is problematic in terms of low solubility in water. In addition, even if such hydrophobic substance is dissolved in an aqueous solution when the solution is prepared, precipitation occurs thereafter over time, and thus, a hydrophobic substance-containing aqueous solution cannot maintain preservation stability. This causes quality loss. Hence, the amount of a hydrophobic substance mixed is limited, and thus, it may be difficult in some cases to obtain a product with satisfactory functions.

In order to solubilize a hydrophobic substance, it is dissolved in and is mixed with an organic solvent with relatively low toxicity, such as ethanol or isopropyl alcohol. However, ethanol is disadvantageous in that it gives unpleasant irritation to a human or the skin of scarp depending on the condition of the scalp, and in that inflammation or the like occurs on the skin of scalp if it is continuously used. Accordingly, an alcohol free type product is desirable.

As techniques of uniformly solubilizing a highly hydrophobic compound in a high concentration in water, an emulsification technique using a surfactant, a microcapsule technique using a hardening agent skillfully, a technique involving solubilization with a polymer or the like, etc. have been developed. In the field of the composition for hair of the present invention, a hydrophobic ingredient is solubilized using a dimethyl diallyl anunonium-acrylamide copolymer, for example (JP Patent Publication (Kokai) No. 5-286840 A (1993)). Moreover, in the case of Kamino 21 manufactured by Kaminomoto Co., Ltd., there is provided an alcohol-free gel type hair growth agent, in which an alkyl methacrylate copolymer is used.

Examples of a compound soluble in water or a water-ethanol liquid include a carboxyvinyl polymer, polyvinyl alcohol, polyvinylpyrrolidone, methylhydroxycellulose, ethylhydroxycellulose, propylhydroxycellulose, alginate, propylene glycol alginate, hyaluronate, veegum, carrageenan, carboxymethyl chitin, polyethyleneimine, acrylic acid resin, and polyacrylate. These compounds have thickening solubilizing ability. Compounds having a hydrophobic group chain as a water-soluble polymer chain, such as vermeulene (an acrylic acid-alkyl methacrylate copolymer), also have the aforementioned functions.

Nevertheless, even using such polymers, in order to set an alcohol concentration at 30% or less, it is necessary to add a water-soluble polymer to maintain solubility. As a result, it has been problematic in that the viscosity of a pharmaceutical agent becomes high, and in that a reduction in exhilaration or stickiness occurs.

Casein is an insoluble protein that constitutes a main body of lactoprotein. Since a hydrophobic portion thereof faces toward outside, casein is likely to form an aggregate. 10 to 100 caseins gather to form a submicelle with a size of approximately 20 nm. Further, 100 to 1,000 caseins gather to form a casein micelle with a size of 90 to 150 nm. Still further, such casein micelles gather to form a micelle aggregate with a size of approximately 500 nm. Accordingly, the size of a casein micelle varies widely, and agglutination occurs when the salts of sodium or magnesium are added to caseins or when the pH thereof is changed to the acidic range.

In recent years, a structure consisting of nanosized particles has been obtained by adsorbing hydrophobic substances into crosslinked casein micelles and remaining them therein. It has been disclosed that a hydrophobic substance is transparently and stably solubilized in a concentration higher than its solubility, namely, in a supersaturated concentration (JP Patent Publication (Kokai) No. 2006-115751 A). However, this publication describes that a protein, and particularly, a casein must be crosslinked, and that a size of 50 nm or less cannot be realized unless casein ingredients are unified. Thus, this technique has considerable restrictions.

A complex coacervation method is a representative method of producing a microcapsule containing a hydrophobic substance (U.S. Patent No. 2800457). In this method, a solvent for solubilizing a hydrophobic substance and a hardening agent that is not suitable for edible use, such as aldehyde, are often used. Thus, with regard to such complex coacervation method involving a complex of a protein and a polysaccharide, there have been disclosed several methods of producing a microcapsule without using a hardening agent that is not suitable for edible use: namely, a method of producing an edible microcapsule **characterized in that** it uses a protein-crosslinking enzyme transglutaminase as a film hardening agent (JP Patent Publication (Kokai) No. 5-292899 A (1993)); a method of producing a microcapsule, which comprises forming a wall film by combining a protein with salts according to a salting-out method and then hardening the wall film with transglutaminase (JP Patent Publication (Kokai) No. 9-248137 A (1997)); and other methods. However, the size of the microcapsule disclosed in the aforementioned publications is several tens of to several hundreds of nanometers in terms of average particle size. Thus, it has not been possible to transparently and stably solubilize a hydrophobic substance even in a supersaturated concentration.

### Disclosure of the Invention

It is an object of the present invention to solve the aforementioned problems of the prior art techniques. That is to say, it is an object of the present invention to provide a composition for hair (e.g. a hair growth agent, a shampoo, a rinse, a hair conditioner, a hair pack, a hair liquid, a hair tonic, a hair spray, or the like), which comprises a hydrophobic ingredient although it has an extremely low alcohol concentration, and which has a low viscosity.

As a result of intensive studies directed towards achieving the aforementioned object, the present inventors have found that protein nanoparticles containing hydrophobic hair active ingredients can be dispersed in a composition for hair with a viscosity of 30 P or less, which comprises 1% to 50% by weight of alcohol and which also comprises a hydrophobic hair active ingredient. The present invention has been completed based on these findings.

The present invention provides a composition for hair having a viscosity of 30 P or less, which comprises 1% to 50% by weight of alcohol and which further comprises a hydrophobic hair active ingredient.

Preferably, the alcohol is ethanol.

Preferably, the hydrophobic hair active ingredient is an ingredient usable for pharmaceutical products, quasi-drug products or cosmetic products, which has a hair growth-promoting effect, a hair growth-stimulating effect, an anti-inflammatory action, or a cell-activating action.

Preferably, the composition for hair of the present invention comprises a protein nanoparticle as a substrate of the hydrophobic hair active ingredient.

Preferably, the composition for hair of the present invention comprises casein nanoparticles prepared by the following steps (a) to (c):
(a) a step of mixing casein or the salt thereof into a basic aqueous medium having a pH value from pH 8 or more to less than pH 11;
(b) a step of adding at least one type of hydrophobic hair active ingredient to the solution obtained in the step (a); and
(c) a step of pouring the solution obtained in the step (b) into an acidic aqueous medium of pH 3.5 to 7.5.

Preferably, the composition for hair of the present invention comprises casein nanoparticles prepared by the following steps (a) to (c):
(a) a step of mixing casein or the salt thereof into a basic aqueous medium having a pH value from pH 8 or more to less than pH 11;
(b) a step of adding at least one type of hydrophobic hair active ingredient to the solution obtained in the step (a); and
(c) a step of decreasing the pH of the solution obtained in the step (b) to a pH value that is apart from the isoelectric point by pH 1 or more, while stirring the solution.

In the present invention, a pharmaceutical product, which comprises a hydrophobic hair active ingredient although it has an extremely low alcohol concentration, and which has a low viscosity can be realized. The pharmaceutical product of the present invention can be produced using casein nanoparticles, for example. That is, since it is not necessary for the composition for hair of the present invention to contain a large amount of ethanol, the level of skin irritation due to such ethanol is low. In addition, the composition for hair of the present invention is advantageous in that, because of its low viscosity, refreshing feeling can be improved, having less stickiness.

### Best Mode for Carrying Out the Invention

The embodiments of the present invention will be further specifically described below.

Examples of an alcohol that can be used for the composition for hair of the present invention include organic solvents having relatively low toxicity, such as ethanol and isopropyl alcohoL Ethanol is particularly preferable. The content of an alcohol in the composition for hair of the present invention is 1% to 50% by weight, preferably 1% to 30% by weight, and more preferably 1% to 20% by weight, relative to the weight of the composition.

The viscosity of the composition, which is appropriately measured at room temperature or a temperature of 25 °C, is 30 P or less, preferably 10 P or less, more preferably 5 P or less, and most preferably 2 P or less.

The composition for hair of the present invention preferably does not comprise a synthetic polymer. The term "synthetic polymer" is used herein to mean a synthetic polymer soluble in water or water-ethanol liquid (a carboxyvinyl polymer, polyvinyl alcohol, polyvinylpyrrolidone, methylhydroxycellulose, ethylhydroxycellulose, propylhydroxycellulose, alginate, propylene glycol alginate, hyaluronate, veegum, carrageenan, carboxymethyl chitin, polyethyleneimine, acrylic acid resin, polyacrylate, etc.), or a compound having a hydrophobic group chain in a water-soluble polymer chain, such as vermeulene (an acrylic acid-alkyl methacrylate copolymer).

A copolymer has 4 types of structures, such as a random copolymer, an alternating copolymer, a periodic copolymer, and a block copolymer. In any of these polymers, solubility as a pharmaceutical product is increased due to its steric structure, while sacrificing high viscosity. Specifically, the aforementioned synthetic polymer includes copolymers obtained by copolymerization of the following three types of components: (A) acrylic acid and/or methacrylic acid; (B) one or two or more types selected from among alkyl acrylate esters and/or alkyl methacrylate esters; and (C) polyoxyalkylene monoalkyl ether and acrylic acid ester, or polyoxyalkylene monoalkyl ether and methacrylic acid ester.

The hydrophobic hair active ingredient used in the present invention is preferably a material that can be used for pharmaceutical products, quasi-drug products, or cosmetic products. Specifically, it can be selected from the group consisting of moisturizing agents, ultraviolet absorbing agents, free-radical-removing agents, antioxidants, anti-inflammatory agents, blood circulation promoters, hair growth agents, hair nutritional supplements, anti-aging agents, collagen synthesis promoters, vitamins, minerals, and amino acids. Specific examples of such agents will be given later.

The composition for hair of the present invention can be achieved by using a protein nanoparticle containing a hair active ingredient therein. The type of protein used in the present invention is not particularly limited. However, a protein having a lysine residue and a glutamine residue is preferable. In addition, such protein having a molecular weight of approximately 10,000 to 1,000,000 is preferably used. The origin of the protein is not particularly limited. However, a human-derived protein is preferably used. Specific examples of a protein that can be used include, but are not limited to, the following compounds according to the present invention: at least one selected from the group consisting of collagen, gelatin, acid-treated gelatin, albumin, ovalbumin, casein, transferrin, globulin, fibroin, fibrin, laminin, fibronectin, and vitronectin. In addition, the origin of the protein is not particularly limited. Thus, any bovine, swine, or fish protein, as well as recombinant protein of any thereof, can be used. Examples of recombinant gelatin that can be used include, but are not limited to, gelatins described in EP1014176 A 2 and US Patent No. 6,992,172. Among them, casein, acid-treated gelatin, collagen, or albumin is preferable. Further, casein is most preferable.

Upon the use of casein according to the present invention, the origin of the casein is not particularly limited. Casein may be milk-derived or bean-derived. Any of α-casein, β-casein, γ-casein, and κ-casein, as well as a mixture of any thereof, can be used. A gene recombinant casein can also be used. Preferably, casein sodium can be used. These caseins can be used alone or in combination of two or more types. The casein used in the present invention may be composed of at least two types selected from α-casein, β-casein and κ-casein. Preferably, the casein comprises α-casein in an amount of 30% or more (more preferably, 40% or more), β-casein in an amount of 15% or more (more preferably, 20% or more), and κ-casein in an amount of 3% or more (more preferably, 5% or more). The α-casein may be composed of α(S1)-casein, and α(S2)-casein, or may be α(S1)-casein alone or α(S2)-casein alone.

Nanoparticles of the present invention can be prepared in accordance with Patent Document: JP Patent Publication (Kokai) No. 6-79168 A (1994); or C. Coester, Journal Microcapsulation, 2000, vol. 17, pp. 187-193, provided that an enzyme is preferably used instead of glutaraldehyde for a crosslinking method.

The protein which constitutes the nanoparticle may be crosslinked or may not be crosslinked. When the protein is crosslinked, any of chemical crosslinking, physical crosslinking or biological crosslinking may be used. When a crosslinking agent is used, the crosslinking agent is preferably added in an amount of 0.1 to 100% by weight to casein or a salt thereof, but the amount of the crosslinking agent is not particularly limited so long as a nanoparticle can be produced.

When chemical crosslinking is carried out, inorganic or organic crosslinking agent can be used as the crosslinking agent. Examples of the inorganic or organic crosslinking agent may include chrome salt (for example, chromium alum, chromium acetate), calcium salt (for example, calcium chloride, calcium hydroxide), aluminum salt (for example, aluminum chloride, aluminum hydroxide), carbodiimides (for example, EDC, WSC, N-hydroxy-5-norbomene-2,3-dicarboxyimide (HONB), N-hydrocysuccinimide (HOSu), dicyclohexylcarbodiimide(DCC)), N-hydroxysuccinimide, phosphorous oxychloride, and vinyl sulfone, but the examples are not limited thereto. The aforementioned crosslinking agent can be used alone or in combination of two or more types thereof. When the crosslinking agent is used, the crosslinking agent is preferably added in an amount of 0.1 to 100% by weight relative to the weight of the protein, and thus crosslinking can be carried out.

Further, a protein to which a reactive group was introduced may be used. Examples of the reactive group includes a photoreactive group (for example, cinnamyl group), a radical generating group (for example, dithiocarbamyl group or a camphoroquinone group), and a vinyl group (for example, styrene group). Further, the protein can be used in combination with a compound which reacts with the reactive group.

When physical crosslinking is carried out, the crosslinking can be carried out by a method of UV irradiation using a monomer having a photoreactive group or a method of generating radicals locally by pulse irradiation so as to carry out crosslinking.

Any enzyme may be used without particular limitation as long as it has been known to have the effect of causing protein crosslinking. Transglutaminase can be preferably used. Transglutaminase may be derived from a mammal or a microorganism. Specific examples thereof include the Activa series by Ajinomoto Co., Inc., commercially available mammalian-derived transglutaminase serving as a reagent, such as guinea pig liver-derived transglutaminase, goat-derived transglutaminase, or rabbit-derived transglutaminase produced by, for example, Oriental Yeast Co., Ltd., Upstate USA Inc., and Biodesign International.

The amount of an enzyme used in a crosslinking treatment according the present invention can be adequately determined depending upon protein type. In general, an enzyme can be added in a weight that is 0.1% to 100% and preferably approximately 1% to 50% of the protein weight.

The duration for an enzymatic crosslinking reaction can be adequately determined depending upon protein type and nanoparticle size. However, in general, the reaction can be carried out for 1 to 72 and preferably 2 to 24 hours.

The temperature for an enzymatic crosslinking reaction can be adequately determined depending upon protein type and nanoparticle size. In general, the reaction can be carried out at 0°C to 80°C and preferably at 25°C to 60°C.

Enzymes used in the present invention may be used alone or in combinations of two or more.

In addition, according to the present invention, the enzymatic crosslinking treatment is preferably carried out in an organic solvent. The organic solvent used herein is preferably an aqueous organic solvent such as ethanol, isopropanol, acetone, or THF. Further, according to the present invention, it is preferable to remove an organic solvent by distillation subsequent to a crosslinking treatment, followed by water dispersion. It is also possible to add water prior to or subsequent to removal of an organic solvent by distillation.

The volume average particle size of the nanoparticles in the present invention is generally 1 to 1000nm, preferably 10 to 1000 nm, more preferably 10 to 200 nm, further preferably 10 to 100 nm, and particularly preferably 20 to 50 nm.

Proteins used in the present invention may be used alone or in combinations of two or more.

It is also possible to add at least one component selected from the group consisting of lipids (e.g., phospholipid), anionic polysaccharides, cationic polysaccharides, anionic proteins, cationic proteins, and cyclodextrin to the nanoparticle of the present invention. The amounts of lipid (e.g. phospholipid), anionic polysaccharide, cationic polysaccharide, anionic protein, cationic protein, and cyclodextrin to be added are not particularly limited. However, they can be added usually in a weight that is 0.1% to 100% by weight of the protein weight. In the case of the composition for hair of the present invention, it is possible to adjust the release rate by changing the ratio of the above components to the protein.

Specific examples of phospholipids that can be used in the present invention include, but are not limited to, the following compounds according to the present invention: phosphatidylcholine (lecithin), phosphatidylethanolamine, phosphatidylserine, phosphatidylinositol, phosphatidylglycerol, diphosphatidylglycerol, and sphingomyelin.

Anionic polysaccharides that can be used in the present invention are polysaccharides having an acidic polar group such as a carboxyl group, a sulfate group, or a phosphate group. Specific examples thereof include, but are not limited to, the following compounds according to the present invention: chondroitin sulfate, dextran sulfate, carboxymethyl cellulose, carboxymethyl dextran, alginic acid, pectin, carrageenan, fucoidan, agaropectin, porphyran, karaya gum, gellan gum, xanthan gum, and hyaluronic acids.

Cationic polysaccharides that can be used in the present invention are polysaccharides having a basic polar group such as an amino group. Examples thereof include, but are not limited to, the following compounds according to the present invention: polysaccharides such as chitin or chitosan, which comprise, as a monosaccharide unit, glucosamine or galactosamine.

Anionic proteins that can be used in the present invention are proteins and lipoproteins having a more basic isoelectric point than the physiological pH. Specific examples thereof include, but are not limited to, the following compounds according to the present invention: poly glutamic acid, polyaspartic acid, lysozyme, cytochrome C, ribonuclease, trypsinogen, chymotrypsinogen, and α-chymotrypsin.

Cationic proteins that can be used in the present invention are proteins and lipoproteins having a more acidic isoelectric point than the physiological pH. Specific examples of such cationic protein include, but are not limited to, the following compounds according to the present invention: polylysine, polyarginine, histone, protamine, and ovalbumin.

According to the present invention, it is possible to use casein nanoparticles (preferably having an average particle size of 10nm or more and less than 100nm) comprising a hydrophobic hair active ingredient which prepared by the following steps (a) to (c):
(a) a step of mixing casein or the salt thereof into a basic aqueous medium having a pH value from pH 8 or more to less than pH 11;
(b) a step of adding at least one type of hydrophobic hair active ingredient to the solution obtained in the step (a); and
(c) a step of pouring the solution obtained in the step (b) into an acidic aqueous medium of pH 3.5 to 7.5.

According to the present invention, it is possible to use casein nanoparticles (preferably having an average particle size of 10nm or more and less than 100nm) comprising a hydrophobic hair active ingredient which are prepared by the following steps (a) to (c):
(a) a step of mixing casein or the salt thereof into a basic aqueous medium having a pH value from pH 8 or more to less than pH 11;
(b) a step of adding at least one type of hydrophobic hair active ingredient to the solution obtained in the step (a); and
(c) a step of decreasing the pH of the solution obtained in the step (b) to a pH value that is apart from the isoelectric point by pH 1 or more, while stirring the solution.

According to the present invention, it is possible to prepare casein nanoparticles of desired sizes. Also, with the use of interaction between a hydrophobic hair active ingredient and a casein hydrophobic domain, it is possible for casein nanoparticles to contain the hydrophobic hair active ingredient. In addition, it was found that such particles remain stable in an aqueous solution.

Further, ionic active ingredient can be contained by a mixed particle of casein or the salt thereof and an ionic polysaccharide or another type ionic protein.

The method for preparing the dispersion of the present invention involves a method wherein casein or salt thereof is mixed with a basic aqueous medium solution and the solution is injected into an acidic aqueous medium, and a method wherein casein or salt thereof is mixed with a basic aqueous medium and the pH of the medium is lowered during stirring, for example.

The method wherein casein or salt thereof is mixed with a basic aqueous medium solution and the solution is injected into an acidic aqueous medium is preferably carried out using a syringe for convenience. However, there is no particular limitation as long as the injection rate, solubility, temperature, and stirring conditions are satisfied. Injection can be carried out usually at an injection rate of 1 mL/min to 100 mL/min. The temperature of the basic aqueous medium can be adequately determined. In general, the temperature is 0°C to 80°C and preferably 25°C to 70°C. The temperature of an aqueous medium can be adequately determined. In general, the temperature can be 0°C to 80°C and preferably 25°C to 60°C. The stirring rate can be adequately determined. However, in general, the stirring rate can be 100 rpm to 3000 rpm and preferably 200 rpm to 2000 rpm.

In the method wherein casein or salt thereof is mixed with a basic aqueous medium and the pH of the medium is lowered during stirring, it is preferable to add acid dropwise for convenience. However, there is no particular limitation as long as solubility, temperature, and stirring conditions are satisfied. The temperature of a basic aqueous medium can be adequately determined. However, in general, the temperature can be 0°C to 80°C and preferably 25°C to 70°C. The stirring rate can be adequately determined. However, in general, the stirring rate can be 100 rpm to 3000 rpm and preferably 200 rpm to 2000 rpm.

The aqueous medium that can be used for the present invention is an aqueous solution or a buffer comprising an organic acid or base or an inorganic acid or base.

Specific examples thereof include, but are not limited to, aqueous solutions comprising: organic acids such as citric acid, ascorbic acid, gluconic acid, carboxylic acid, tartaric acid, succinic acid, acetic acid, phthalic acid, trifluoroacetic acid, morpholinoethanesulfonic acid, and 2-[4-(2-hydroxyethyl)-1-piperazinyl]ethmemlfonic acid; organic bases such as tris (hydroxymethyl), aminomethane, and ammonia; inorganic acids such as hydrochloric acid, perchloric acid, and carbonic acid; and inorganic bases such as sodium phosphate, potassium phosphate, calcium hydroxide, sodium hydroxide, potassium hydroxide, and magnesium hydroxide.

The concentration of an aqueous medium used in the present invention is preferably approximately 10 mM to 1 M, and more preferably approximately 20 mM to 200 mM.

The pH of a basic aqueous medium used in the present invention is preferably 8 or more, more preferably 8 to 12, and further preferably 10 to 12. When the pH is excessively high, there is concern regarding hydrolysis or risks in handling. Thus, the pH is preferably in the above range.

According to the present invention, the temperature at which casein is mixed with a basic aqueous medium at a pH of 8 or more is preferably 0°C to 80°C, more preferably 10°C to 70°C, and further preferably 30°C to 70°C.

The pH of an acidic aqueous medium used in the present invention is preferably 3.5 to 7.5 and more preferably 5 to 7.5. When the pH does not fall in the above range, the particle size tends to become large.

The type of the hair active ingredient used in the present invention is not particularly limited. It can be selected from the group consisting of cosmetic ingredients, quasi-drug ingredients, and pharmaceutical ingredients, for example. In the present invention, specific examples of a hair active ingredient contained in a protein nanoparticle include hydrophobic ingredients selected from the group consisting of moisturizing agents, ultraviolet absorbing agents, free-radical-removing agents, antioxidants, anti-inflammatory agents, blood circulation promoters, hair growth agents, hair nutritional supplements, anti-aging agents, collagen synthesis promoters, vitamins, minerals, and amino acids, which are as listed below. In the present invention, it was found that a hair active ingredient can be incorporated into a casein nanoparticle by utilizing the interaction between a fat-soluble hair active ingredient and a casein hydrophobic portion. Further, it was also found that such particles are stably present in an aqueous solution. Such a fat-soluble substance preferably has a Clog P of greater than 0, and more preferably has a Clog P of 1 or greater.

Examples of a moisturizing agent include agar, diglycerin, distearyldimonium hectorite, butylene glycol, polyethylene glycol, propylene glycol, hexylene glycol, *Coix lachrma-jobi* extract, vaseline, urea, hyaluronic acid, ceramide, Lipidure, isoflavone, amino acid, collagen, mucopolysaccharide, fucoidan, lactoferrin, sorbitol, chitin/chitosan, malic acid, glucuronic acid, placenta extract, seaweed extract, moutan cortex extract, sweet tea extract, hypericum extract, coleus extract, *Euonymus japonicus* extract, safflower extract, Rosa rugosa flower extract, *Polyporus sclerotium* extract, hawthorn extract, rosemary extract, duke extract, chamomile extract, *Lamium album* extract, *Litchi Chinensis* extract, *Achillea millefolium* extract, aloe extract, marronnier extract, *Thujopsis dolabrata* extract, Fucus extract, Osmoin extract, oat bran extract, tuberosa polysaccharide, *Cordyceps sinensis* (plant worm) extract, barley extract, orange extract, *Rehmannia glutinosa* extract, zanthoxylumb extract, and *Coix lachrma-jobi* extract. In addition, in the case of casein nanoparticles, casein itself has moisture retention capacity.

Examples of an ultraviolet protecting agent include homomenthyl salicylate, 4-methoxycinnamic acid-2-ethylhexyl, 2-hydroxy-4-methoxy benzophenone, 2-hydroxy-4-methoxy benzophenone sulfonic acid, 2-hydroxy-4-methoxy benzophenone sodium sulfonate, 4-t-butyl-4'-methoxy-dibenzoylmethane, titanium oxide, and zinc oxide.

Examples of a free-radical-removing agent include superoxide dismutase (SOD), mannitol, carotenoids such as beta carotene, astaxanthin, rutin and derivatives thereof, bilirubin, cholesterol, tryptophan, histidine, quercetin, quercitrin, catechin, catechin derivatives, gallic acid, gallic acid derivatives, *Scutellariae radix* extract, ginkgo extract, *Saxifraga stolonifera* (strawberry geranium) extract, melissa extract, *Geranium thunbergii* extract, moutan cortex extract, parsley extract, tormentilla extract, *Momordica grosvenori* extract, seaweed extract, "*Yashajitsu*" (*Alnus firma Sieb. et Zucc*.) extract, and Lycii cortex extract.

Examples of an antioxidant include carotenes, retinoic acid, retinol, vitamin C and derivatives thereof, kinetin, astaxanthin, tretinoin, vitamin E and derivatives thereof, sesamin, α-lipoic acid, coenzyme Q10, flavonoids, ezythorbic acid, gallic acid propyl, BHT (di-n-butylhydroxytoluene), BHA (butylhydroxyanisole), *Engelhardtia chrysolepis Hance* extract, soybean extract, black tea extract, green tea extract, and *Rosae multiflorae fructus* extract.

Examples of an anti-inflammatory agent include: compounds selected from the group consisting of azulene, guaiazulene, diphenhydramine hydrochloride, hydrocortisone acetate, predonisolone, glycyrrhizic acid, glycyrrhetic acid, mefenamic acid, phenylbutazone, indomethacin, ibuprofen and ketoprofen, the derivatives thereof, and the salts thereof; and plant extracts selected from the group consisting of *Scutellariae radix* extract, *Artemisia capillaris* extract, balloonflower (*Platycodon grandiflorus*) extract, *Armeniacae semen* extract, gardenia extract, *sasa veitchii* extract, gentiana extract, comfrey extract, white birch extract, mallow extract, *Persicae semen* extract, peach leaf extract, and *Eriobotryae folium* extract.

Examples of a blood circulation promoter that can be selected include nicotinic acid, *Swertia japonica* extract, γ-oxazole, alkoxycarbonylpyridine N-oxide, carpronium chloride, and acetylcholine or derivatives thereof.

Examples of a hair growth agent include glycyrhetic acid or derivatives thereof, glycyrrhizic acid or derivatives thereof, hinokitiol, minoxidil and analogs thereof, adenosine, vitamin E and derivatives thereof, vitamin C derivatives, 6-benzyl aminopurine, nicotinic acid benzyl, nicotinic acid tocopherol, nicotinic acid β-butoxy ester, isopropyl methylphenol, pentadecanoic acid and derivatives thereof, cephalathin, finasteride, t-flavanone, and pantothenyl ethyl ether. Among them, hinokitiol and minoxidil or analogs thereof are particularly preferable.

Known ingredients can be used also as hair nutritional supplements, anti-aging agents, collagen synthesis promoters, vitamins, minerals, and amino acids.

The above hair active ingredients may be used singly or in combinations of two or more types.

Further, it was found that a particle mixture of protein and ionic polysaccharide or another ionic protein contains an ionic hair active ingredient.

The composition for hair of the present invention comprises preferably 0.01% to 50% by weight and most preferably 0.1% to 10% by weight protein nanoparticles.

The composition for hair of the present invention contains a hair active ingredient in a weight that is preferably 0.1% to 100% and more preferably 0.1% to 50% of the protein weight.

According to the present invention, a hair active ingredient may be added during or after formation of protein nanoparticles.

Further, the composition for hair of the present invention may comprise, as an additive, a hair active ingredient. Specific examples of such a hair active ingredient that serves as an additive include, but are not limited to, pantothenic acid, panthenol, licorice extract, *Lepisorus thunbergianus* extract, *Sophorae Radix* (sophora root) extract, *Swertia japonica* extract, capsicum extract, *Ampelopsis cantoniensis var. grossedentata* extract, carrot extract, *Taraxacum mongolicum Hand.-Mazz*. extract, tree peony extract, and mandarin orange extract.

The composition for hair of the present invention may further comprise additives that are able to provide specific effects. The type of such additive is not particularly limited. One or more types selected from the group consisting of a microbicide, an anti-inflammatory agent, a blood circulation promoter, a freshener, a moisturizing agent, a softening agent, a transdermal-absorption-promoting agent, an antioxidant, a coloring agent, a thickener, an aroma chemical, and a pH adjuster can be used as an additive(s).

Specific examples of a microbicide that can be used in the present invention will be given below. However, the type of such a microbicide is not particularly limited, as long as it exhibits microbicidal effects. Such a microbicide can be appropriately selected from cosmetic ingredients, quasi drug ingredients, or pharmaceutical ingredients. In addition, the term "microbicide" is used to mean that a certain product has antibacterial action because of its wide range of antibacterial spectrum.

A microbicide is preferably an ionic substance or a fat-soluble substance, and is particularly preferably a fat-soluble substance. The type of such a microbicide includes a synthetic product, a plant extract, and the like.

Specific examples of such a microbicide include acrinol, sulfur, calcium gluconate, chlorhexidine gluconate, sulfamine, mercurochrome, lactoferrin or a hydrolyzed product thereof, an alkyldiaminoethylglycine chloride solution, triclosan, sodium hypochlorite, chloramine T, chloride of lime, an iodine compound, iodoform, sorbic acid or a salt thereof, propionic acid or a salt thereof, salicylic acid, resorcin, dehydroacetic acid, parahydroxybenzoic acid esters, undecylenic acid, thiamine dilaurylsulfate, thiamine dilaurylnitrate, phenol, 2,2,4-trichlor-2-hydroxyphenol, cresol, p-chlorophenol, p-chloro-m-xylenol, p-chloro-m-cresol, thymol, phenethyl alcohol, o-phenylphenol, Irgasan CH3565, Halocarban, hexachlorophene, chlorohexidine, ethanol, methanol, isopropyl alcohol, benzyl alcohol, ethylene glycol, propylene glycol, 2-phenoxyethanol, 1,2-pentanediol, zinc pyrithione, chlorobutanol, isopropylmethylphenol, nonionic surfactants (polyoxyethylene lauryl ether, polyoxyethylene nonyl phenyl ether, polyoxyethylene octyl phenyl ether, etc.), amphoteric surfactants, anionic surfactants (sodium lauryl sulfate, lauroylsarcosine potassium, etc.), cationic surfactants (cetyltrimethylammonium bromide, benzalkonium chloride, benzethonium chloride, methylrosanilinium chloride), formaldehyde, hexamine, brilliant green, malachite green, crystal violet, germal, photosensitizing dye 101, photosensitizing dye 201, photosensitizing dye 401, an N-long chain acyl basic amino acid derivative and an acid-added salt thereof, zinc oxide, hinokitiol, Sophora root, and propolis. Preferred examples include benzoic acid, sodium benzoate, methyl parahydroxybenzoate, ethyl parahydroxybenzoate, propyl parahydroxybenzoate, butyl parahydroxybenzoate, potassium sorbate, sodium sorbate, sorbic acid, sodium dehydroacetate, hydrogen peroxide, formic acid, ethyl formate, sodium hypochlorite, propionic acid, sodium propionate, calcium propionate, pectin degradation products, polylysine, phenol, isopropylmethyl phenol, orthophenylphenol, phenoxyethanol, resorcin, thymol, thiram, and tea tree oil. More preferred examples include phenoxyethanol, thymol, cineol, isopropylmethylphenol, methyl parahydroxybenzoate, and ethyl parahydroxybenzoate.

The anti-inflammatory agent used in the present invention is preferably an ionic substance or a fat-soluble substance. Specific examples of such an antiflammatory agent that can be used in the present invention include: compounds selected from the group consisting of azulene, guaiazulene, diphenhydramine hydrochloride, hydrocortisone acetate, predonisolone, glycyrrhizic acid, glycyrrhetic acid, mefenamic acid, phenylbutazone, indomethacin, ibuprofen and ketoprofen, the derivatives thereof, and the salts thereof; plant extracts selected from the group consisting of *Scutellariae radix* extract, *Artemisia capillaris* extract, balloonflower (*Platycodon grandiflorus*) extract, *Armeniacae semen* extract, gardenia extract, *Sasa veitchii* extract, gentiana extract, comfrey extract, white birch extract, mallow extract, *Persicae semen* extract, peach leaf extract, and *Eriobotryae folium* extract; proteins; polysaccharides; and animal extracts.

Specific examples of a blood circulation promoter that can be used in the present invention will be given below. The type of such a blood circulation promoter is not particularly limited, as long as it has an effect of promoting blood circulation. The term "promotion of blood circulation" is used herein to mean an action to increase the volume of blood flow by vasodilatation caused by rexation or reinforcement of capillary vessel, stimulation to capillary vessel, and an increase of temperature. The blood circulation promoter used in the present invention is preferably an ionic substance or a fat-soluble substance. In addition, examples of a blood circulation promoter that can be used in the present invention include a synthetic product, a plant extract, vitamins, and sugars.

Examples of such a synthetic product include minoxidil that is well known as a good agent for hypertension, finasteride that is well kwon as a good agent for prostatic hypertrophy, and carpronium chloride that is well known as a good agent for alopecia areata.

Examples of such a plant extract include swertia herb extract obtained from the rhizome and stolon of gentianaceous plants, carrot extract obtained from the rhizome and stolon of araliaceous plants, Sophora flavescens extract obtained from the rhizome and stolon of fabaceous plants, Mentha herb extract obtained from the leaves of peppermint or the like, cepharanthin that is the alkanoid of menispermaceous plants, capsicum tincture obtained from capsicum, ginger tincture obtained from ganger, and garlic extract obtained from garlic.

Examples of such vitamins include vitamin B, vitamin E, and the derivative thereof. Specific examples of vitamin E and the derivative thereof include tocopherol, tocopherol acetate, and tocopherol nicotinate. Such tocopherol is preferably natural α-tocopherol. In addition, examples of vitamin B and the derivative thereof include nicotinic acid, nicotinamide, and benzyl nicotinate. Vitamins vary widely, ranging from hydrophilic substances to hydrophobic substances.

As sugars, mucopolysaccharides are preferable. A specific example is heparin having ability to suppress blood coagulation.

Preferred examples of the blood circulation promoter used in the present invention include a tocopherol derivative, a nicotinic acid derivative, cepharanthin, finasteride, minoxidil, and swertia herb extract.

Examples of a freshener used in the present invention include menthol, camphor, mint, and eucalyptus oil. Of these, the use of menthol is particularly preferable, and the use of L-menthol is further preferable.

Specific examples of a moisturizing agent that can be used in the present invention include, but are not limited to, agar, diglycerin, distearyldimonium hectorite, butylene glycol, polyethylene glycol, propylene glycol, hexylene glycol, *Coix lachrma-jobi* extract, vaseline, urea, hyaluronic acid, ceramide, Lipidure, isoflavone, amino acid, collagen, mucopolysaccharide, fucoidan, lactoferrin, sorbitol, chitin/chitosan, malic acid, glucuronic acid, placenta extract, seaweed extract, moutan cortex extract, sweet tea extract, hypericum extract, coleus extract, *Euonymus japonicus* extract, safflower extract, Rosa rugosa flower extract, *Polyporus sclerotium* extract, hawthorn extract, rosemary extract, duke extract, chamomile extract, *Lamium album* extract, *Litchi Chinensis* extract, *Achillea millefolium* extract, aloe extract, marronnier extract, *Thujopsis dolabrata* extract, Fucus extract, Osmoin extract, oat bran extract, tuberosa polysaccharide, *Cordyceps sinensis* (plant worm) extract, barley extract, orange extract, *Rehmannia glutinosa* extract, zanthoxylumb extract, and *Coix lachrma-jobi* extract.

Specific examples of a softening agent that can be used in the present invention include, but are not limited to, glycerin, mineral oil, and emollient ingredients (e.g., isopropyl isostearate, polyglyceryl isostearate, isotridecyl isononanoate, octyl isononanoate, oleic acid, glyceryl oleate, cacao butter, cholesterol, mixed fatty acid triglyceride, dioctyl succinate, sucrose acetate stearate, cyclopentasiloxane, sucrose distearate, octyl palmitate, octyl hydroxystearate, arachidyl behenate, sucrose polybehenate, polymethylsilsesquioxane, myristyl alcohol, cetyl myristate, myristyl myristate, and hexyl laurate, etc.).

Specific examples of a transdermal-absorption-promoting agent that can be used in the present invention include, but are not limited to, ethanol, isopropyl myristate, citric acid, squalane, oleic acid, menthol, N-methyl-2-pyrrolidone, diethyl adipate, diisopropyl adipate, diethyl sebacate, diisopropyl sebacate, isopropyl palmitate, isopropyl oleate, octyldodecyl oleate, isostearyl alcohol, 2-octyl dodecanol, urea, vegetable oil, and animal oil.

Specific examples of an antioxidant that can be used in the present invention include, but are not limited to, vitamin A, retinoic acid, retinol, retinol acetate, retinol palmitate, retinyl acetate, retinyl palmitate, tocopheryl retinoate, vitamin C and the derivative thereof, kinetin, β-carotene, astaxanthin, lutein, lycopene, tretinoin, vitamin E, α-lipoic acid, coenzyme Q10, polyphenol, SOD, and phytic acid.

Specific examples of a coloring agent that can be used in the present invention include, but are not limited to, krill pigment, orange dye, cacao dye, kaoline, carmines, ultramarine blue, cochineal dye, chrome oxide, iron oxide, titanium dioxide, tar dye, and chlorophyll.

Specific examples of a thickener that can be used in the present invention include, but are not limited to, quince seed, carrageenan, gum arabic, karaya gum, xanthan gum, gellan gum, tamarind gum, locust bean gum, gum traganth, pectin, starch, cyclodextrin, methylcellulose, ethylcellulose, carboxymemylcellulose, and sodium alginate.

Specific examples of an aroma chemical that can be used in the present invention include, but are not limited to, musk, acacia oil, anise oil, ylang ylang oil, cinnamon oil, jasmine oil, sweet orange oil, spearmint oil, geranium oil, thyme oil, neroli oil, mentha oil, hinoki (Japanese cypress) oil, fennel oil, peppermint oil, bergamot oil, lime oil, lavender oil, lemon oil, lemongrass oil, rose oil, rosewood oil, anisaldehyde, geraniol, citral, civetone, muscone, limonene, and vanillin.

Specific examples of a pH adjuster that can be used in the present invention include, but are not limited to, sodium citrate, sodium acetate, sodium hydroxide, potassium hydroxide, phosphoric acid, succinic acid, and the salts thereof.

As the composition for hair of the present invention, it is preferable that the aforementioned nanoparticles be dispersed in an aqueous substrate and be then used. The dosage form of a dispersion product is not particularly limited. Examples of the dosage form include a liquid for external use, an aerosol, a lotion, a tonic, a liniment, an emulsion, a suspension, a saturant, a foaming agent, a cosmetic lotion, a face pack, and a sheet-like external preparation.

Ingredients contained in the composition for hair of the present invention are not particularly limited. Such ingredients may be selected from cosmetic ingredients or pharmaceutical ingredients, for example. Examples of such a cosmetic ingredient include a moisturizing agent, a hair growth agent, a hair restorer, a hair growing stimulant, an anti-white hair agent, an anti-aging agent, an antioxidant, a collagen synthesis promoter, vitamin, an aroma chemical, a coloring agent, an anhidrotic, a cooling agent, and a warming agent. Examples of such a pharmaceutical ingredient include a hair growth agent, a hair restorer, a hair growing stimulant, an antibiotic, an anti-inflammatory agent, an antiallergic agent, a hormone agent, a therapeutic agent for skin disease, an antifungal agent, an antipruritic agent, a sweater, an anhidrotic, an angiotonic, a vasodilator, vitamin, polypeptide, hormone, and a skin softener. The aforementioned active ingredients may be used singly or in combination of two or more types.

The composition for hair of the present invention can be used as a hair growth agent or a cosmetic product for hair having a single or multiple effect(s), depending on ingredients contained therein.

Examples of the dosage form of the composition for hair of the present invention include, but are not limited to, a liquid formulation for external use, a fomentation, an embrocation, a bathing agent, a bath additive, a disinfectant, ointment, gel, cream, paste, an adhesive skin patch, a plaster, a wound-surface-covering agent, a wound-surface-covering gauze, a hemostatic, an adhesive, an adhesive tape, an adhesive tape for transdermal absorption, a wound protective agent, aerosol, lotion, tonic, liniment, emulsion, suspension, a saturant, a tincture, powders, a foaming agent, skin lotion, massage cream, nourishing cream, face pack, a sheet-like external preparation, a cosmetic product for makeup, a skin coloring agent for external use, a cosmetic skin adhesive, shampoo, rinse, hair conditioner, hair pack, hair liquid, hair tonic, hair spray, a permanent wave composition, hair dye, body soap, soap, a bath agent, a sun care product (e.g., sunscreen, sun tanning oil, and after-sun lotion), and fragrance.

The method for administering the composition for hair of the present invention is not particularly limited. For instance, it can be administered by directly applying it to the scalp.

The dose of the composition for hair of the present invention can be adequately determined depending on the type and amount of a hair active ingredient and on the body weight and condition of a user, for example. The dose for single administration is generally approximately 1 µg to 50 mg/cm² and preferably 2.5 µg to 10 mg/cm².

The present invention will be further specifically described in the following examples. However, these examples are not intended to limit the scope of the present invention.

### Examples

### Example 1:

30 mg of milk-derived casein Na (manufactured by Wako Pure Chemical Industries, Ltd.) was mixed with 1 mL of a 50 mM phosphate buffer (pH 9). 1.7 mg of glycyrrhetic acid (manufactured by Wako Pure Chemical Industries, Ltd.) was dissolved in 0.25 mL of ethanol. While stirring, the glycyrrhetic acid solution was added dropwise to the casein solution. Thereafter, using a microsyringe, 1 mL of the mixed solution was poured into 10 mL of a 200 mM phosphate buffer solution (pH 5) under stirring conditions consisting of an outside temperature of 40°C and 800 rpm, so as to obtain a water dispersion of casein nanoparticles containing glycyrrhetic acid. The volume average particle size of the above particles was measured using "ZetasizerNano" manufactured by Sysmex, and it was found to be 18 nm.

### Examples 2 to 9 and Comparative examples 1 to 5

Hereafter, each of the hydrophobic ingredients as shown in Tables 1 and 2 was dissolved in ethanol and was mixed therewith in the same manner as described above, so as to prepare each sample. In addition, the average particle size of the particles of each sample was measured using "Zetasizer Nano" manufactured by Sysmex. Further the viscosity of the sample was measured using a VISCOMATE VM-10A shaking type viscometer manufactured by CBC Materials Co. The results are shown in Tables 1 and 2.

**Table 1**

| Additive compound | Comparative. Example 1 | Comparative. Example 2 | Comparative. Example 3 | Comparative. Example 4 | Comparative. Example 5 | Example 1 | Example 2 |
|---|---|---|---|---|---|---|---|
| Casein sodium | | | | | | 2 | 2 |
| Alkyl methacrylate copolymer | | | 4 | | | | |
| Sodium citrate | | | | | 1 | 1 | |
| Tocopherol acetate | | | | 0.2 | | | 0.1 |
| Glycyrrhetic acid | 0.03 | 0.03 | 0.1 | | 0.03 | 0.03 | |
| Ethanol | 90 | 1.5 | 0 | 95 | 1.5 | 1.5 | 1.3 |
| Viscosity (distilled water: 1.15) | 1.2 | 1.3 | 62.4 | 1.4 | 1.4 | 1.6 | 1.6 |
| Solubility of ingredients | Dispersed | Precipitated | Dispersed | Dispersed | Precipitated | Dispersed | Dispersed |

**Table 2**

| Additive compound | Example9 | Example 3 | Example 4 | Example 5 | Example 6 | Example 7 | Example 8 |
|---|---|---|---|---|---|---|---|
| Casein sodium | 1 | 1 | 1 | 2 | 2 | 1 | 1 |
| Sodium citrate | | 1 | 1 | 1 | 1 | 1 | |
| Sodium phosphate | | | | | | | 0.8 |
| Glycyrrhetic acid | 0.01 | 0.01 | 0.01 | 0.01 | | 0.02 | |
| Dipotassium glycyrrhizinate | | | | | 0.01 | 0.005 | 0.01 |
| Pantothenyl ethyl ether | | 0.2 | 0.2 | 0.2 | 0.2 | 0.1 | |
| Pantophenyl ether alcohol | 0.2 | | | | | 0.1 | 0.2 |
| Benzyl nicotinate | | | | | | | 0.01 |
| Nicotinamide | 0.015 | 0.015 | 0.015 | 0.015 | 0.015 | 0.03 | |
| L-menthol | 0.008 | | 0.008 | | | 0.012 | 0.008 |
| Hinokitiol | | | | 0.035 | 0.035 | | |
| Methylparaben | 0.1 | | 0.15 | 0.15 | 0.15 | | 0.13 |
| Phenoxyethanol | 0.05 | 0.5 | | | | | |
| Ascorbic phosphate sodium | | 0.05 | | 0.05 | 0.05 | | |
| Ascorbic phosphate magnesium | | | | | | 0.05 | |
| Ascorbic sulfate disodium | 0.05 | | 0.05 | | | | 0.1 |
| Ethanol | 1.5 | 1.5 | 1.7 | 1.5 | 1.5 | 2 | 1.5 |
| Viscosity(distilled water: 1.15) | 1.8 | 1.9 | 1.5 | 1.7 | 1.6 | 1.4 | 1.4 |
| Solubility of ingredients | Dispersed | Dispersed | Dispersed | Dispersed | Dispersed | Dispersed | Dispersed |
| Particle size | 21 | 20.4 | 21.3 | 21.1 | 19.8 | 21.3 | 20.8 |

As shown in Tables 1 and 2 above, according to the present invention, a hydrophobic ingredient, glycyrrhetic acid, could be converted to an aqueous dispersion solution at a low viscosity and in a low ethanol concentration.

## Claims

1. A composition for hair having a viscosity of 30 P or less, which comprises 1% to 50% by weight of alcohol and which further comprises a hydrophobic hair active ingredient.

2. The composition for hair according to claim 1, wherein the alcohol is ethanol.

3. The composition for hair according to claim 1, wherein the hydrophobic hair active ingredient is an ingredient usable for pharmaceutical products, quasi-drug products or cosmetic products, which has a hair growth-promoting effect, a hair growth-stimulating effect, an anti-inflammatory action, or a cell-activating action.

4. The composition for hair according to claim 1, which comprises a protein nanoparticle as a substrate of the hydrophobic hair active ingredient.

5. The composition for hair according to claim 1, which comprises casein nanoparticles prepared by the following steps (a) to (c):
(a) a step of mixing casein or the salt thereof into a basic aqueous medium haying a pH value from pH 8 or more to less than pH 11;
(b) a step of adding at least one type of hydrophobic hair active ingredient to the solution obtained in the step (a); and
(c) a step of pouring the solution obtained in the step (b) into an acidic aqueous medium of pH 3.5 to 7.5.

6. The composition for hair according to claim 1, which comprises casein nanoparticles prepared by the following steps (a) to (c):
(a) a step of mixing casein or the salt thereof into a basic aqueous medium having a pH value from pH 8 or more to less than pH 11;
(b) a step of adding at least one type of hydrophobic hair active ingredient to the solution obtained in the step (a); and
(c) a step of decreasing the pH of the solution obtained in the step (b) to a pH value that is apart from the isoelectric point by pH 1 or more, while stirring the solution.
